# EUROPEAN PATENT SPECIFICATION

(11) **EP 3 349 011 B1**
(45) Date of publication and mention of the grant of the patent: **23.02.2022**
(21) Application number: 16844478.4
(22) Date of filing: 09.09.2016
(51) Int. Cl.: G01N 33/574, C12Q 1/02

(54) **LIVER CANCER TEST METHOD**
TESTVERFAHREN FÜR LEBERKREBS
PROCÉDÉ DE TEST DE CANCER DU FOIE

(30) Priority: 10.09.2015 JP 2015178402
(43) Date of publication of application: 18.07.2018
(73) Proprietor: Sekisui Medical Co., Ltd., Tokyo 103-0027 (JP); Social Welfare Organization Saiseikai Imperial Gift Foundation, Inc., Tokyo 108-0073 (JP); Miyazaki, Toru, Tokyo 112-0003 (JP)
(72) Inventor: MIYAZAKI, Toru, Tokyo 112-0003 (JP); OKANOUE, Takeshi, Suita-shi Osaka 564-0013 (JP); KOYAMA, Noriyuki, Tokyo 162-0812 (JP)
(74) Representative: J A Kemp LLP
(86) International application number: PCT/JP2016/076562
(87) International publication number: WO 2017/043617

(56) References cited:
- WO-A1-2011/145725
- WO-A1-2017/022315
- US-A1- 2015 094 268
- US-A1- 2015 094 268
- TOMOKO YAMAZAKI ET AL: "Circulating AIM as an Indicator of Liver Damage and Hepatocellular Carcinoma in Humans", PLOS ONE, vol. 9, no. 10, 10 October 2014 (2014-10-10), page e109123, XP055368997, DOI: 10.1371/journal.pone.0109123
- SATOKO ARAI ET AL: "Apoptosis inhibitor of macrophage protein enhances intraluminal debris clearance and ameliorates acute kidney injury in mice", NATURE MEDICINE, vol. 22, no. 2, 4 January 2016 (2016-01-04), pages 183-193, XP055362476, New York ISSN: 1078-8956, DOI: 10.1038/nm.4012
- TOMOKO YAMAZAKI ET AL: "A proteolytic modification of AIM promotes its renal excretion", SCIENTIFIC REPORTS, vol. 6, no. 1, 1 December 2016 (2016-12-01), XP055410649, DOI: 10.1038/srep38762
- KOYAMA NORIYUKI ET AL: "Activation of apoptosis inhibitor of macrophage is a sensitive diagnostic marker for NASH-associated hepatocellular carcinoma", JOURNAL OF GASTROENTEROLOGY, SPRINGER JAPAN KK, JP, vol. 53, no. 6, 30 October 2017 (2017-10-30), pages 770-779, XP036513196, ISSN: 0944-1174, DOI: 10.1007/S00535-017-1398-Y [retrieved on 2017-10-30]
- MIYAZAKI TORU ET AL: "AIM associated with the IgM pentamer: attackers on stand-by at aircraft carrier", CELLULAR & MOLECULAR IMMUNOLOGY, CHINESE SOCIETY OF IMMUNOLOGY, CH, vol. 15, no. 6, 29 January 2018 (2018-01-29), pages 563-574, XP036562408, ISSN: 1672-7681, DOI: 10.1038/CMI.2017.141 [retrieved on 2018-01-29]
- MERA, KUMIKO ET AL.: 'Serum levels of apoptosis inhibitor of macrophage are associated with hepatic fibrosis in patients with chronic hepatitis C' BMC GASTROENTEROLOGY vol. 14, no. 27, 2014, pages 1 - 10, XP021177479
- YAMAZAKI, TOMOKO ET AL.: 'Circulating AIM as an indicator of liver damage and hepatocellular carcinoma in humans' PLOS ONE vol. 9, no. 10, 2014, pages 1 - 12, XP055368997
- GRAY, JOE ET AL.: 'A proteomic strategy to identify novel serum biomarkers for liver cirrhosis and hepatocellular cancer in individuals with fatty liver disease' BMC CANCER vol. 9, no. 271, 2009, pages 1 - 11, XP021057649
- ARAI, SATOKO ET AL.: 'Obesity-associated autoantibody production requires AIM to retain the immunoglobulin M immune complex on follicular dendritic cells' CELL REPORTS vol. 3, 2013, pages 11 87 - 1198, XP009172535
- GANGADHARAN, BEVIN ET AL.: 'Novel serum biomarker candidates for liver fibrosis in hepatitis C patients' CLINICAL CHEMISTRY vol. 53, no. 10, 2007, pages 1792 - 1799, XP055018487
- MIYAZAKI, TORU ET AL.: 'Increased susceptibility of thymocytes to apoptosis in mice lacking AIM, a novel murine macrophage-derived soluble factor belonging to the scavenger receptor cysteine-rich domain superfamily' JOURNAL OF EXPERIMENTAL MEDICINE vol. 189, no. 2, 1999, pages 413 - 422, XP008149323

## Description

### Technical Field

The present invention relates to a method for examining liver cancer. Specifically, the present invention relates to a method for examining liver cancer, comprising quantifying free AIM in a biological sample of a test subject.

### Background Art

AIM (apoptosis inhibitor of macrophage; also referred to as CD5L, api6, or Spα) is a secretory protein having a molecular weight of approximately 50 kDa that is specifically produced by tissue macrophage (Non-Patent Literature 1). AIM has a structure of tandemly connected three scavenger receptor cysteine-rich (SRCR) domains which are specific sequences containing a large number of cysteine residues, and it is thought to have a compact spherical conformation due to each cysteine residue being bound to one another by disulfide bonds within each domain.

AIM is an adhesive protein, and various molecules have been reported as binding partners thereof. For example, it is known to recognize the pathogen-associated molecular patterns (PAMPs) of bacteria and fungus such as lipoteichoic acid (LTA) or lipopolysaccharide (LPS), and have the ability to aggregate bacteria (Non-Patent Literature 2). There are also many cells in the body that bind AIM on their surface or incorporate it into the cell, and it has been reported that AIM is incorporated into macrophage itself which is the producer cell, or that it is incorporated by endocytosis via scavenger receptor CD36 in fat cells to induce fat degradation (Non-Patent Literature 3).

It has also been long known that AIM binds to IgM in blood (Non-Patent Literature 4). In recent years, it has been reported that binding to IgM is closely involved in order for AIMs to be not excreted into urine and to exist stably in blood, and that nearly all AIMs form a complex with IgM in the blood and they are rarely present in a non-bound form (Non-Patent Literature 5, 6).

Liver cancer (hepatocellular carcinoma; HCC) is a disease that is poor in effective therapeutic methods, and is one of the top causes of death among carcinomas. Moreover, there has currently been an increase in non-alcoholic fatty liver diseases (NAFLD) together with lifestyle-related diseases. Among these, since the number of patients who have progressed from a state of showing only fatty liver (non-alcoholic fatty liver; NAFL) to non-alcoholic steatohepatitis (NASH) accompanied by inflammation and fibrillization is significantly increasing, it is deemed certain that the number of patients who develop HCC derived from NASH will increase in the future (Non-Patent Literature 7). In light of this background, there is a significant need related to developments of a diagnosis marker of HCC and an effective therapeutic method that makes use thereof.

There have been several reports thus far on the relationship between AIM and liver cancer. For example, a method for measuring AIM concentration in a sample collected from a test subject has been reported as a method for diagnosing or examining AIM-associated disease including liver cancer (Patent Literature 1).

Moreover, in a method for diagnosing liver diseases including liver cancer, there is reported a method for diagnosing liver disease comprising determining that said test subject has liver disease or is highly likely to contract liver disease when the AIM concentration in a sample from a test subject is lower than the AIM concentration in a sample from a healthy individual (Patent Literature 2).

Further, it has been reported that blood AIM value of HCC patients is significantly high compared to healthy adults (Non-Patent Literature 8), circulating AIM level may be used as diagnostic and/or prognostic marker of HCC (Non-Patent Literature 9), CD5L was significantly increased in many hepatic cirrhosis individuals with HCC (Non-Patent Literature 10), increase in expression of CD5L in the HCC stage was found compared to hepatic cirrhosis in HCV-infected patients (Non-Patent Literature 11), etc.

In addition, prophylactic or therapeutic agents for hepatic disease, containing AIM or a partial peptide thereof, or a nucleic acid containing a base sequence encoding the same are disclosed in Patent Literature 3, together with a method of screening for a hepatic disease comprising using on animal obtained by loading a non-human mammal deficient in AIM expression with a high fat diet.

However, there have been no reports thus far regarding the relationship between non-bound AIM that has not formed a complex with other binding partners, in particular IgM, and liver cancer. There have also been no reports on a method that can discriminate HCC and other liver diseases including NAFL or NASH with high precision.

### Citation List

### [Patent Literatures]

[Patent Literature 1] International Publication WO2011/145725 A1
[Patent Literature 2] International Publication WO2013/162021 A1
[Patent Literature 3] US 2015/094268

### [Non-Patent Literatures]

[Non-Patent Literature 1] Miyazaki T et al. Increased Susceptibility of Thymocytes to Apoptosis in Mice Lacking AIM, a Novel Murine Macrophage-derived Soluble Factor Belonging to the Scavenger Receptor Cysteine-rich Domain Superfamily. J Exp Med 189:413-422, 1999
[Non-Patent Literature 2] Sarrias MR et al. A role for human Sp alpha as a pattern recognition receptor. J Biol Chem 280:35391-35398, 2005
[Non-Patent Literature 3] Kurokawa J et al. Macrophage-derived AIM is endocytosed into adipocytes and decreases lipid Ddroplets via inhibition of fatty acid synthase activity. Cell Metab 11:479-492, 2010
[Non-Patent Literature 4] Tissot JD et al. IgM are associated to Sp alpha (CD5 antigen-like). Electrophoresis 23:1203-1206, 2002.
[Non-Patent Literature 5] Miyazaki T et al., "Latest Knowledge on Macrophage-derived Protein AIM - Their Diverse Functions and Association with Lifestyle-related Diseases -", Nippon Rinsho, Vol. 71, No. 9 (2013-9), pages 1681-1689
[Non-Patent Literature 6] Miyazaki T et al. Obesity-Associated Autoantibody Production Requires AIM to Retain the Immunoglobulin M Immune Complex on Follicular Dendritic Cells, Cell Reports 3, 1187-1198, April 25, 2013
[Non-Patent Literature 7] Ascha MS et al. The incidence and risk factors of hepatocellular carcinoma in patients with nonalcoholic steatohepatitis. Hepatology 51:1972-1978, 2010
[Non-Patent Literature 8] Miyazaki T, Suppression of Fatty Liver Hepatocellular Carcinoma by Steatolysic protein AIM, Oxidative Stress, and Liver Disease <Vol. 10> 99-106, Published March 27, 2015 [Non-Patent Literature 9] Yamazaki T et al. "Circulating AIM as an Indicator of Liver Damage and Hepatocellular Carcinoma in Humans" PLOS ONE, 9 (10); e109123, 2014
[Non-Patent Literature 10] Gray J et al. "A proteomic strategy to identify novel serum biomarkers for liver cirrhosis and hepatocellular cancer in individuals with fatty liver disease" BMC Cancer 2009, 9:271
[Non-Patent Literature 11] Sarvari J et al. "Differentially Expressed Proteins in Chronic Active Hepatitis, Cirrhosis, and HCC Related to HCV Infection in Comparison With HBV Infection: A proteomics study" Hepatitis Monthly. 2013 Jul; 13 (7):e8351

### Summary of the Invention

### Problems to be Solved by the Invention

The object of the present invention is to provide a method for examining liver cancer that is superior in sensitivity and specificity.

### Means for Solving the Problems

As a result of repeated extensive investigation by the present inventors to solve the above problem, it was found that free AIM is significantly increased in a biological sample of a liver cancer test subject. As it was thought that free AIM could normally be barely confirmed in blood, it was surprising that, among other things, significant increase of free AIM in the serum of liver cancer test subject was confirmed.

The present invention is based on the above knowledge.

In other words, in one embodiment, the present invention relates to a method for examining liver cancer comprising a step of quantifying an amount of free AIM in a serum, plasma or whole blood sample derived from a test subject, wherein an increased amount compared to a control healthy individual indicates liver cancer, wherein free AIM means AIM protein that is not in complex with other binding partners such as IgM..

In one embodiment, the method of the present invention is characterized in that said quantification is one by immunoassay. For example, in the method of the present invention, said quantification can be one that is performed by contacting a biological sample with an antibody that specifically binds to free AIM. Moreover, in one embodiment of the present invention, said quantification can be measured by ELISA.

In one embodiment, the method of the present invention is characterized in that said quantification is performed without exposing the serum, blood plasma, or whole blood to a protein denaturing condition and to a reductive condition.

In one embodiment, the method of the present invention can be one that further comprises a step of quantifying total AIM in a biological sample derived from a test subject. In the method of the present invention, the amount of free AIM can be calculated as the ratio against total AIM.

In the present invention, said liver cancer may be liver cancer caused by non-alcoholic steatohepatitis (NASH).

The present invention is defined by the appended claims whose subject-matter is reproduced below.
(1) A method for examining liver cancer comprising a step of quantifying an amount of free AIM in a serum, plasma or whole blood sample derived from a test subject wherein an increased amount compared to a control healthy individual indicates liver cancer, wherein free AIM means AIM protein that is not in complex with other binding partners such as IgM.
(2) The method according to (1), wherein said liver cancer is liver cancer caused by non-alcoholic steatohepatitis (NASH).
(3) The method according to (1) or (2), wherein said quantification is one by immunoassay.
(4) The method according to (3), wherein said quantification is performed by contacting a biological sample with an antibody that specifically binds to free AIM.
(5) The method according to (3) or (4), wherein said quantification is measured by ELISA.
(6) The method according to any of (2) to (5), characterized in that said quantification is performed without exposing the serum, blood plasma, or whole blood to a protein denaturing condition and to a reductive condition.
(7) The method according to (1) further comprising a step of quantifying total AIM in a biological sample derived from a test subject.
(8) The method according to (7) further comprising a step of calculating the ratio of free AIM and total AIM.

### Effects of the Invention

According to the present invention, there is provided a method for detecting liver cancer or a method for assisting diagnosis of liver cancer that is superior in sensitivity and specificity.

### Brief Description of the Drawings

Figure 1 shows the result of Western blot analysis with an anti-AIM antibody in the serum of healthy individuals, NAFL patients, NASH patients, and NASH liver cancer patients (HCC). In the figures, (A) shows the result in which the serum was exposed to a reductive condition, and (B) shows the result under a non-reductive condition.
Figure 2 shows the result of detecting free AIM with Human AIM ELISA kit (CY-8080; from CircuLex) in the serum of healthy individuals, NAFL patients, NASH patients, and NASH liver cancer patients (HCC).
Figure 3 shows the result of quantifying free AIM at each fibrillization stage in the serum of NASH patients (NASH) and NASH liver cancer patients (HCC).
Figure 4 shows the result of quantifying Total AIM at each fibrillization stage in the serum of NASH patients (NASH) and NASH liver cancer patients (HCC).
Figure 5 shows a scatter diagram generated based on the amount of free AIM and Total AIM quantified in the serum of NAFL patients, NASH patients (collectively non-HCC), and NASH liver cancer patients (HCC).
Figure 6 shows the result of ROC analysis carried out with NAFL patients, NASH patients (collectively non-HCC), and NASH liver cancer patients (HCC).

### Description of Embodiments

The embodiments of the present invention will now be specifically described.

The method for examining liver cancer according to the present invention comprises a step of quantifying free AIM in a biological sample derived from a test subject. As described above, AIM is a secretory protein having a molecular weight of approximately 50 kDa that is specifically produced by tissue macrophage, and is known to be expressed in humans and many mammals other than humans as well as birds. It is also known that in order for AIM to exist stably in blood without being excreted into urine, nearly all AIMs form a complex with IgM and they are rarely present as a non-bound form (Non-Patent Literatures 5 and 6). There are also suggestions that the possibility of having or contracting a liver disease is high when the AIM concentration in a sample from a test subject is a lower value (Patent Literature 2). In spite of this knowledge, it has now been found that free AIM in a biological sample is significantly increased in liver cancer test subjects. Accordingly, by detecting or quantifying free AIM in a biological sample derived from a test subject, liver cancer can be detected with good detection sensitivity and specificity in the aforementioned test subject. "Free AIM" as used herein refers to non-bound AIM in a biological sample that has not formed a complex with other binding partners of AIM, in particular IgM. In contrast, "total AIM" is a generic term for "free AIM" and AIM that has formed a complex with other binding partners (such as IgM) (hereinbelow also referred to as complex AIM), and "detect or measure total AIM" refers to detecting or measuring both free AIM and complex AIM in a biological sample.

"Examining liver cancer" or "assisting diagnosis of liver cancer" as used herein means inspecting a sample collected from a test subject in order to obtain information necessary for diagnosis. Accordingly, the method of the present invention may be carried out not only at a medical institution but at e.g. an examination company.

In the present invention, the test subject is not particularly limited but includes e.g. a test subject having a risk of developing or is suspected of having developed liver cancer. The test subject also includes a test subject who has already developed liver cancer, in which case the present invention can be carried out with the objective to determine the prognosis of such test subject or to determine the effect of a particular liver cancer therapy. The test subject typically refers to a human, but may also include other animals including e.g. other primates, rodents, a dog, a cat, a horse, a sheep, a pig, and the like.

In the present invention, liver cancer refers to a malignant tumor that develops in the liver, and means a hepatocellular carcinoma that undergoes primary development in the liver. In one embodiment, the liver cancer is a liver cancer caused by non-alcoholic steatohepatitis (NASH).

In yet another aspect, the present disclosure can further comprise a step of administering a therapy or treatment of liver cancer to a test subject determined to have a risk of developing or to be suspected of having developed liver cancer by the method of the present invention for examining liver cancer. However, the invention does not encompass such a step.

In the present invention, the sample is serum, plasma or whole blood.

It is particularly preferred to employ serum. Moreover, said body fluid can be a body fluid itself that is collected from a test subject, or it may be those subjected to treatments such as dilution or concentration ordinarily performed on collected body fluids. Note that the person who collects and prepares the biological sample derived from a test subject employed in the present invention may be the same or different from the person who performs the steps of the present invention. Moreover, the biological sample derived from a test subject employed in the present invention may be collected or prepared at the time of carrying out the present invention, or collected or prepared in advance and stored.

The method for detecting or quantifying free AIM is not particularly restricted as long as it is a method that can detect and discriminate between free AIM and complex AIM, and an ordinarily employed protein detection method can be applied. It is also preferred that such a protein detection method is a method that can quantify or semi-quantitatively measure free AIM. Such a method can include, but is not limited to, a method employing an antibody that binds to free AIM, molecular sieve chromatography, ion exchange chromatography, mass spectrometry, and the like.

In the present invention, the device employed for detecting free AIM is not particularly restricted, and can be appropriately selected depending on the method for detecting or measuring free AIM. This can include, specifically, e.g. an HPLC instrument, a mass spectrometry instrument, an electrophoresis instrument (such as a capillary electrophoresis device), an automatic or semi-automatic enzyme immunoassay instrument, a cell washer, an automatic or semi-automatic chemiluminescent immunoassay instrument, a luminescence measuring device, an automatic or semi-automatic electrochemiluminescent immunoassay instrument, an optical measuring device, a plate reader, a CCD camera, an automatic or semi-automatic fluorescent immunoassay instrument, a fluorescence measuring device, an automatic or semi-automatic radioimmunoassay instrument, a liquid scintillation counter, a Coulter counter, a surface plasmon measuring device, a blotting device, a densitometer, and the like.

In the present invention, in terms of detection sensitivity, specificity and convenience, it is preferred to use a method that employs an antibody that binds to free AIM (also referred to herein as an "anti-free AIM antibody"), e.g. an immunoassay that employs an anti-free AIM antibody. The anti-free AIM antibody is not particularly limited as long as it is an antibody that can recognize and bind AIM.

For the anti-free AIM antibody, both monoclonal and polyclonal antibodies can be produced according to a well-known method. A monoclonal antibody can for example be obtained by isolating antibody-producing cells from a non-human mammal immunized with a free AIM or free AIM fragment, fusing them with myeloma cells etc. to produce hybridomas, and purifying the antibody produced by this hybridoma. Moreover, a polyclonal antibody can be obtained from the serum of an animal immunized with a free AIM or free AIM fragment. A free AIM fragment is a partial peptide of free AIM, and an anti-free AIM fragment antibody recognizes free AIM. Moreover, the immunogen includes, but is not limited to, e.g. a free AIM or free AIM fragment of primates such as a human or a monkey, rodents such as a rat or a mouse, a dog, a cat, a horse, a sheep, a pig, and the like.

An "antibody" as used herein refers to a full length immunoglobulin molecule that exists in nature or is produced by a genetic recombination technology, or an immunologically active fragment of an immunoglobulin molecule such as an antibody fragment. Unless particularly noted, the antibody of the present invention includes any type, class, or subclass, e.g. includes IgG, IgE, IgM, IgD, IgA, IgY, IgG₁, IgG₂, IgG₃, IgG₄, IgA₁, IgA₂, and the like. These antibodies can be produced with a conventional technology, and may be a polyclonal antibody or a monoclonal antibody. An antibody fragment includes F(ab')₂, F(ab)₂, Fab', Fab, Fv, scFv, and the like. An antibody fragment can be produced by e.g. genetic recombination technology employing an encoding nucleic acid, or it can also be produced by cleaving a full length antibody with an enzyme. In the present invention, it is preferred that the anti-free AIM antibody is a monoclonal antibody.

In the present invention, examples of an anti-free AIM antibody can include e.g. AIM-CL-6 (Accession No: NITE BP-1092) and AIM-CL-7 (Accession No: NITE BP-1093) deposited at the National Institute of Technology and Evaluation Patent Microorganism Depository.

It is preferred that the anti-free AIM antibody used in the present invention is an antibody that specifically binds to free AIM. "Specific binding" refers to a binding that is different in measurement from a non-specific interaction, and further herein to an antibody that has higher biding affinity to free AIM than to AIM that has formed a complex with IgM. In one aspect of the present invention, "specific binding" may be shown by e.g. an antibody with a Kd of at least approximately 10⁻⁴ M, or at least approximately 10⁻⁵ M, or at least approximately 10⁻⁶ M, or at least approximately 10⁻⁷ M, or at least approximately 10⁻⁸ M, or at least approximately 10⁻⁹ M, or at least approximately 10⁻¹⁰ M, or at least approximately 10⁻¹¹ M, or at least approximately 10⁻¹² M, or more against a free AIM. In another aspect, "specific binding" means binding to free AIM without substantial binding to polypeptides other than free AIM, including AIM that has formed a complex with IgM.

A method commonly used by those skilled in the art can be employed as the immunoassay in the present invention, although a method or a condition where free AIM and complex AIM are detected or quantified with discrimination should be selected. For example, in the method of the present invention, it is preferred to perform fractionation of the biological sample based on molecular weight as necessary upon detection or quantification with an anti-free AIM antibody so that free AIM and complex AIM are detected with discrimination. It is also preferred to perform detection or quantification with an anti-free AIM antibody after separating free AIM from complex AIM. Moreover, in the method of the present invention, care should be taken so that said biological sample is not exposed to a protein denaturing condition or reductive condition in which the bound state of the AIM that has formed a complex with IgM gets dissolved. Such a protein denaturing condition or reductive condition is well-known to those skilled in the art as a method or condition that destroys or inhibits protein conformation or protein-protein binding.

An immunoassay employs a detectably labeled anti-free AIM antibody, or an antibody against a detectably labeled anti-free AIM antibody (secondary antibody). Depending on the method of labeling the antibody, they are classified into enzyme immunoassay (EIA or ELISA), radioimmunoassay (RIA), fluorescent immunoassay (FIA), fluorescence polarization immunoassay (FPIA), chemiluminescent immunoassay (CLIA), electrochemiluminescent immunoassay (ECLIA), and the like, any of which can be employed in the method of the present invention.

An antibody labeled with an enzyme such as peroxidase and alkaline phosphatase is employed in the ELISA method, with a radioactive material such as ¹²⁵I, ¹³¹I, ³⁵S, and ³H in the RIA method, with a fluorescent substance such as fluorescein isothiocyanate, rhodamine, dansyl chloride, phycoerythrin, tetramethylrhodamine isothiocyanate, and near-infrared fluorescent material in the FPIA method, and with a luminescent substance such as luciferase, luciferin, and aequorin in the CLIA method. In addition, an antibody labeled with a nanoparticle such as gold colloid and quantum dot can also be detected.

In an immunoassay, anti-free AIM antibody can also be labeled with biotin, and bound to avidin or streptavidin labeled with e.g. an enzyme to detect and measure free AIM.

Among immunoassays, the ELISA method which employs an enzyme label is preferred in that it can conveniently and rapidly measure a target.

In the ELISA method, the sandwich method can for example be employed. The anti-free AIM antibody is fixed onto a solid phase carrier, an appropriately treated biological sample is added and allowed to react, and then an anti-free AIM antibody that recognizes another epitope labeled with an enzyme is further added and allowed to react. After washing, the mixture is reacted with an enzyme substrate for color development, and absorbance can be measured to determine the concentration of free AIM. Moreover, after allowing the anti-free AIM antibody fixed onto the solid phase carrier and the free AIM in the biological sample to react, an unlabeled free AIM antibody (primary antibody) is added, and an antibody against this unlabeled antibody (secondary antibody) may be labeled with an enzyme and further added.

When the enzyme is a peroxidase, 3,3'-diaminobenzidine (DAB), 3,3'5,5'-tetramethylbenzidine (TMB), o-phenylenediamine (OPD), and the like can be employed as the enzyme substrate, and when it is an alkaline phosphatase, p-nitropheny phosphate (NPP) and the like can be employed.

In the above immunoassay, ECLIA is also preferred as a method for measuring protein with high sensitivity. In the ECLIA method, the anti-free AIM antibody is fixed to microbeads, an appropriately treated biological sample is added and allowed to react, and then an anti-free AIM antibody that recognizes another epitope labeled with an electrochemiluminescent substance is added and allowed to react. After washing the microbeads, the microbeads are captured onto an electrode, electrical energy is applied to allow luminescence, and the amount of luminescence can be measured to determine the concentration of free AIM.

In the above immunoassay, the aggregation method is also preferred as a method that can conveniently detect a trace amount of protein. An aggregation method includes e.g. the latex aggregation method having an antibody bound to a latex particle.

When a latex particle is bound to an anti-free AIM antibody and mixed with a biological sample, the antibody-bound latex particles aggregate if free AIM is present. The aggregated mass is then quantified by irradiating near-infrared light on the sample and measuring absorbance (turbidimetry) or scattered light (nephelometry) to determine the concentration of antigen.

In the present invention, a kit that can specifically detect free AIM can be employed for quantifying free AIM. The aforementioned kit can include e.g. a commercially available kit, and this can be employed for detecting or quantifying free AIM according to the instruction of the aforementioned kit. Such a kit can include, but is not limited to, Human AIM ELISA kit (CY-8080; from CircuLex) and the like.

The method of the present invention may further comprise, in addition to detecting or quantifying free AIM, a step of quantifying total AIM in a biological sample derived from a test subject. For example, in NASH liver cancer patients, depending on the liver fibrillization stage, no significant increase of total AIM in the biological sample is seen while significant increase of free AIM is seen. Accordingly, for example by further evaluating the amount of total AIM, liver cancer can be examined with higher precision and specificity. In one embodiment of the present invention, the amount of free AIM is calculated as the ratio against total AIM.

Detection or quantification of total AIM can be performed by any protein detection method conventional to those skilled in the art, and such a method can include, but is not limited to, a method that employs an antibody that binds to AIM, molecular sieve chromatography, ion exchange chromatography, mass spectrometry, and the like. In one embodiment of the present invention, in terms of specificity and convenience, it is preferred to measure by an immunoassay employing any antibody that binds to AIM, e.g. ELISA. Moreover, in the present invention, the device employed for detecting total AIM is not particularly restricted, and can be appropriately selected depending on the method for detecting or measuring total AIM.

In the present invention, a kit that can specifically detect total AIM can be employed for detecting or quantifying total AIM. The aforementioned kit can include e.g. a commercially available kit, and this can be employed for detecting or quantifying total AIM according to the instruction of the aforementioned kit. Such a kit can include, but is not limited to, Human AIM ELISA kit (KK901; from Trans Genic) and the like.

In one embodiment of the present invention, detection or quantification of total AIM can be performed by converting the complex AIM in the biological sample into free AIM by exposing a biological sample derived from a test subject having free AIM detected or quantified to a protein denaturing condition or a reductive condition, and then quantifying the total amount of free AIM.

In one embodiment of the present invention, detection or quantification of free AIM and detection or quantification of total AIM can also be performed simultaneously in one measurement. Such an embodiment can include a method that combines the above method that employs an antibody that binds to AIM with molecular sieve chromatography. An antibody that binds to AIM is an antibody that can recognize both free AIM and AIM that has formed a complex with other binding partners, and is also referred to herein as an "anti-AIM antibody". Accordingly, the anti-AIM antibody may be an anti-free AIM antibody and/or an antibody that can recognize AIM that has formed a complex with other binding partners. In one embodiment, the anti-AIM antibody is an anti-free AIM antibody that recognizes a region on free AIM that is different from the region that forms a complex with other binding partners. When the anti-AIM antibody and a biological sample are mixed, the anti-AIM antibody binds to each of complex AIM or free AIM in case where complex AIM and free AIM are present in the said sample. When the said mixed sample is subjected to molecular sieve chromatography, since the free AIM having anti-AIM antibody bound thereto and the complex AIM having anti-AIM antibody bound thereto are separated and eluted at retention times according to their respective molecular weights, the ratio of free AIM, complex AIM, total AIM, and free AIM against total AIM can be measured simultaneously in one measurement by measuring the peak deriving from free AIM and the peak deriving from complex AIM. The method such as above of separating and measuring a bound substance of antibody and antigen by chromatography is well-known by e.g. Japanese Published Unexamined Patent Application Publication No. H 2-028557 and the like. There are also no restrictions on the labeling of an anti-AIM antibody or application of chromatography other than molecular sieve, and they can be appropriately selected and used in combination in light of the description herein on the detection or quantification of free AIM and the detection or quantification of total AIM.

The method of the present invention can further comprise a step of determining liver cancer based on the amount of free AIM in a biological sample from said test subject. The amount of free AIM in the present invention is not particularly limited, as long as it is the concentration of said free AIM contained in a biological sample from said test subject, or a quantitative value or a semiquantitative value corresponding thereto. Examples of the amount of free AIM in the present invention can include the measurement value of directly measuring the amount of said free AIM detected, the measurement value of indirectly measuring the amount of said free AIM detected via detection of a label, and the like.

In the determination step of the method of the present invention, by comparing the amount of free AIM in a biological sample from a test subject with the amount of free AIM in a biological sample of a control healthy individual as the reference value (cutoff value), liver cancer can be determined if the amount of free AIM in a biological sample derived from said test subject is larger.

The control healthy individual means an individual who is proved in advance to not have developed liver cancer. Moreover, the amount of free AIM is larger refers to that the amount of free AIM from the test subject is larger than the reference value (cutoff value) set to discriminate a healthy individual from a liver cancer patient.

Said reference value can be set for example by subjecting the amount of free AIM in a biological sample of a liver cancer patient and the amount of free AIM in a biological sample of a control group of healthy individuals to ROC analysis (Receiver Operating Characteristic analysis) and the like. ROC analysis is for example an analysis method that can evaluate the detecting and diagnosing ability of a method for examining an illness, such as an analysis method that was described in The Japan Society for Clinical Laboratory Automation Journal "Evaluation Manual for Diagnostic Utility of Clinical Examination", Ver. 1.3 (2004.9.1), Vol. 29 Suppl. 1 (Whole Number 154) (Published Spe. 1, 2004).

The reference value can also be set as e.g. the sum of the average value of detection levels of healthy individuals and two- or three-folds of the standard deviation value, and can further be appropriately set as a value that fulfills sensitivity (detection rate) and specificity (how low the rate of false positives is) in a balanced manner.

For example, this includes setting 1.681 (1.12 + 0.187 * 3) µg/mL as the sum value of the average value of healthy individuals and three-folds of the standard deviation, or 1.494 (1.12 + 0.187 * 2) µg/mL as the sum value of the average value of healthy individuals and two-folds of the standard deviation (based on the measurement value by Human AIM ELISA kit (CY-8080, from CircuLex)) by applying the above protocol to the free AIM measurement value of healthy individual serum (1.12 ± 0.187 µg/mL) and the free AIM measurement value of NASH liver cancer patient serum (2.90 ± 1.375 µg/mL) as described in Example 3 below, and further adjusting it to a value that fulfills sensitivity and specificity in a balanced manner.

As another aspect of the above determination step, for example, patients can also be grouped according to pathology etc., and measurement results of free AIM between each patient group can be compared. An example of this can include comparison of NAFL and NASH patients group (non-HCC patient group) with NASH liver cancer (HCC patient group) as described in the Examples below. Moreover, the cutoff value for such a case can include 1.6 µg/mL or more (based on the measurement value by Human AIM ELISA kit (CY-8080, from CircuLex)) as described in the Examples below.

In another aspect, the present disclosure relates to a kit for examining or assisting diagnosis of liver cancer. In one aspect of the present disclosure , the kit according to the present disclosure is a kit for performing the above-described method for examining liver cancer according to the present invention, and comprises an anti-free AIM antibody. In one aspect of the kit of the present disclosure , an anti-free AIM antibody is an antibody that specifically binds to free AIM.

The aforementioned kit for examining or assisting diagnosis comprises a reagent and a device necessary for measuring the concentration of free AIM in the biological sample by an immunoassay that utilizes the antigen antibody reaction between free AIM and an anti-free AIM antibody.

In one aspect, the kit of the present disclosure is for measuring the concentration of free AIM by a sandwich ELISA method, and comprises a microtiter plate; an anti-free AIM antibody for capturing; an anti-free AIM antibody labeled with an alkaline phosphatase or a peroxidase; and an alkaline phosphatase substrate (such as NPP) or a peroxidase substrate (such as DAB, TMB, and microtiter plate OPD). The capturing antibody and the labeled antibody recognize different epitopes. In such a kit, the capturing antibody is first fixed onto a microtiter plate, an appropriately treated and diluted biological sample is added thereto and then incubated, and the sample is removed and washed. Subsequently, the labeled antibody is added and then incubated, and the substrate is added for color development. By measuring the color development with e.g. a microtiter plate reader, the concentration of free AIM can be determined.

In another aspect, the kit of the present disclosure is for measuring the concentration of free AIM by the sandwich ELISA method that uses a secondary antibody, and comprises a microtiter plate; an anti-free AIM antibody for capturing; an anti-free AIM antibody as the primary antibody; an antibody against an anti-free AIM antibody labeled with an alkaline phosphatase or a peroxidase as the secondary antibody; and an alkaline phosphatase substrate (such as NPP) or a peroxidase substrate (such as DAB, TMB, and OPD). The capturing antibody and the primary antibody recognize different epitopes. In such a kit, the capturing antibody is first fixed onto a microtiter plate, an appropriately treated and diluted biological sample is added thereto and then incubated, and the sample is removed and washed. Subsequently, the primary antibody is added followed by incubation and washing, an enzyme-labeled secondary antibody is further added and incubated, and then the substrate is added for color development. By measuring the color development with e.g. a microtiter plate reader, the concentration of free AIM can be determined. By employing a secondary antibody, the reaction is amplified and detection sensitivity can be increased.

It is also preferred that the kit of the present disclosure further comprises the necessary buffer, enzyme reaction quenching solution, microplate reader, instruction, and the like.

The labeled antibody is not limited to an enzyme-labeled antibody, and may be an antibody labeled with a radioactive material (such as ²⁵I, ¹³¹I, ³⁵S, and ³H), a fluorescent substance (such as fluorescein isothiocyanate, rhodamine, dansyl chloride, phycoerythrin, tetramethylrhodamine isothiocyanate, and near-infrared fluorescent material), a luminescent substance (such as luciferase, luciferin, and aequorin), a nanoparticle (gold colloid and quantum dot), and the like. It is also possible to employ a biotinylated antibody as the labeled antibody and add a labeled avidin or streptavidin to the kit.

Another aspect of the diagnostic kit of the present disclosure also includes those for measuring the concentration of free AIM with high sensitivity by the ECLIA method. This kit comprises microbeads having anti-free AIM antibodies solid-phased thereon, an electrochemiluminescent substance (such as ruthenium)-labeled anti-free AIM antibody, and the like. In such a kit, an appropriately treated biological sample is added to microbeads having anti-free AIM antibodies solid-phased thereon and allowed to react, and then the sample is removed and washed. Subsequently, an anti-free AIM antibody that recognizes another epitope labeled with an electrochemiluminescent substance is added and allowed to react. After washing the microbeads, the microbeads are captured onto an electrode, electrical energy is applied to allow luminescence, and the amount of luminescence can be measured to determine the concentration of free AIM.

Yet another aspect of the diagnostic kit of the present disclosure also includes those for measuring the concentration of free AIM by the latex aggregation method. This kit comprises anti-free AIM antibody-sensitized latex, wherein the biological sample and the anti-free AIM antibody are mixed and the agglomerate is quantified by an optical method. It is also preferred that the kit comprises an aggregation reaction plate for visualizing the aggregation reaction.

The present invention will now be more specifically described with Examples. The present invention is not to be limited in any way by the Examples shown below.

### Examples

### Example 1: Preparation of Mouse Anti-human AIM Monoclonal Antibody <Animal Sensitization>

As the antigen, full length human rAIM (2 mg/ml) was mixed with an equal amount of TiterMax Gold (G-1, Funakoshi) to prepare an emulsion. Two 8 week old female Balb/c mice (from Charles River) were employed as immune animals, and 50 µL of the antigen solution was administered to the posterior plantar region. A similar administration was performed after 2 weeks, and 50 µg of the antigen solution was administered to the posterior plantar region after another 2 weeks or more in order to prepare for cell fusion 3 days later.

### <Myeloma Cell>

Mouse P3U1 was employed as the myeloma cell, and a medium which was RPMI1640 (11875-119 GIBCO) supplemented with glutamine and pyruvic acid and having FBS (S1560 from BWT) added to be 10% was employed as the proliferation culture. As antibiotics, penicillin and streptomycin were added at appropriate amounts.

<Cell Fusion>

From a mouse from which cardiac blood was collected under anesthesia, popliteal lymph nodes were aseptically resected, placed on a beaker with a #200 mesh, and a cell suspension was prepared by thrusting with a silicon stick. The cells were subjected to centrifugation wash two times in RPMI1640, and then the number of cells was counted. Myeloma cells in the logarithmic growth phase state were collected by centrifugation and washed, after which the ratio of lymphocytes to myeloma cells was adjusted to be 5 to 1, and mixed centrifugation was performed. Cell fusion was performed with PEG1500 (783641, Roche). In other words, cell pellets were allowed to react with 1 mL of PEG solution over 3 minutes, this was serially diluted and washed by centrifugation, the medium was added and 200 µL each was placed in fifteen 96-well plates, and subjected to 1 week of culture. As the medium, a myeloma cell medium supplemented with HAT Supplement (21060-017, GIBCO) and FBS concentration adjusted to 15% was employed.

### <Collection of Mouse Peritoneal Fluid>

Cells stored in a frozen state were thawed, proliferation culture was performed, then 1 × 10⁷ cells were administered to the peritoneal cavity of a nude mouse (BALB/cAJcl-nu/nu, CLEA Japan) having 0.5 ml of pristane (42-002, Cosmo Bio) intraperitoneally administered 1 week or more beforehand, and 4 - 12 ml of peritoneal fluid was obtained after about 2 weeks later. Solids were removed by centrifugation treatment and then this was stored in a frozen state.

### Example 2: Detection of Free AIM in Srerum Specific for NASH Liver Cancer (HCC)

The serum of 4 healthy individual cases, 6 NAFL patient cases, 6 NASH patient cases, and 14 NASH liver cancer patient cases (HCC) were subjected to agarose gel electrophoresis under reductive or non-reductive condition, and Western blot was performed with an anti-AIM antibody.

In a reductive condition, the patient serum was mixed with a sample buffer comprising SDS and mercaptoethanol, allowed to be reduced at 99°C for 5 minutes, and then added to e-PAGEL (from ATTO) gel and separated by SDS-PAGE method. In a non-reductive condition, the patient serum was mixed with a sample buffer that does not comprise SDS and mercaptoethanol, and separated by Native-PAGE method. After electrophoresis, the protein was transferred onto a PVDF membrane (Immobilon, from Millipore), and primary antibody reaction was performed with the mouse anti-human AIM monoclonal antibody prepared in Example 1 at 4°C overnight. HRP-bound antimouse IgG antibody was employed as the secondary antibody, Luminata Forte Western HRP Substrate (from Millipore) was employed as the detection reagent, and signal detection was carried out with Image Quant LAS 4000 (GE Healthcare).

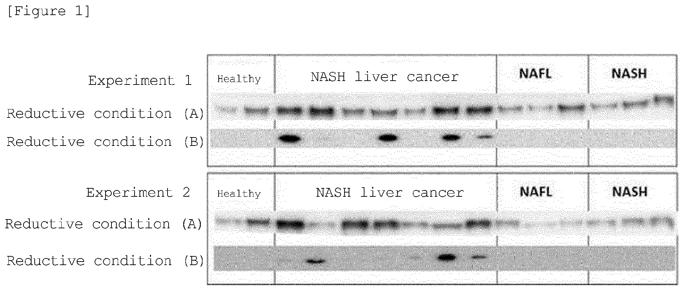

Under a reductive condition (A), a single band of free AIM was seen since all AIM molecules are disengaged from IgM. Under a non-reductive condition (B), free AIM band was not detected in healthy individuals, NAFL patients, and NASH patients since AIM is not disengaged from IgM, but free AIM band was detected in NASH liver cancer patients (HCC). Similar results were confirmed in Experiments 1 and 2 which used different patient serums (Figure 1). It was also confirmed by this experiment that the mouse anti-human AIM monoclonal antibody prepared in Example 1 binds to AIM.

### Example 3: Quantification of Free AIM in Serum

Human AIM ELISA kit (CY-8080, from CircuLex) that quantifies free AIM was employed to measure the concentration of free AIM in the serum of 10 healthy individual cases, 42 NAFL patient cases, 135 NASH patient cases, and 26 NASH liver cancer patient cases (HCC). Measurement was carried out according to the instruction of the kit. Man-Whittney U test was employed for statistical analysis, and p < 0.05 was considered to be significantly different.

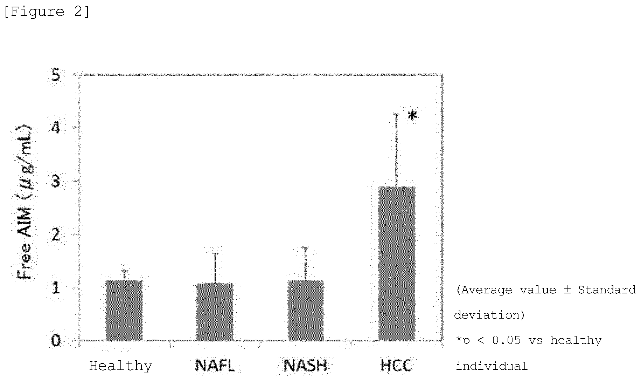

As a result, the concentration of free AIM in NAFL and NASH patients was 1.07 ± 0.568 µg/mL and 1.12 ± 0.632 µg/mL, respectively, and did not show difference to healthy individuals (1.12 ± 0.187 µg/mL), but NASH liver cancer patients (HCC) showed significantly high free AIM at 2.90 ± 1.375 µg/mL (Figure 2).

### Example 4: Quantification of Free AIM and Total AIM in Each Fibrillization Stage of Liver

The aforementioned Human AIM ELISA kit (CY-8080, from CircuLex) that quantifies free AIM was employed to measure the concentration of free AIM in the serum of 135 NASH patient cases and 26 NASH liver cancer patient cases (HCC).

Further, ELISA which quantifies total AIM was employed to measure the concentration of total AIM in patient serum. Upon measurement, a standard material having the concentration valued by BCA method was employed. In the aforementioned ELISA, the mouse anti-human AIM monoclonal antibody was solid-phased onto a plate and the patient serum was added according to conventional method. After washing, this was reacted with an HRP-labeled mouse anti-human AIM monoclonal antibody and subjected to color development with an HRP substrate. All of the anti-AIM antibodies employed were antibodies prepared according to the method described in Example 1 which are antibodies that recognize both free AIM and complex AIM and each having different epitopes.

Since increase in total AIM is reported along with progression of liver fibrillization, the patients were divided into three groups of fibrillization stages 1 and 2 (F1-F2), stage 3 (F3), and stage 4 (F4) for analysis according to Brunt's classification (Am J Gastroenterol. 1999 Sep; 94 (9): 2467-2674). Among NASH patients, with the exception of 27 cases where fibrillization was not seen, the number of cases for each of NASH patients and NASH liver cancer patients (HCC) analyzed was 71 and 4 cases for F1-F2, 30 and 10 cases for F3, and 7 and 12 cases for F4.

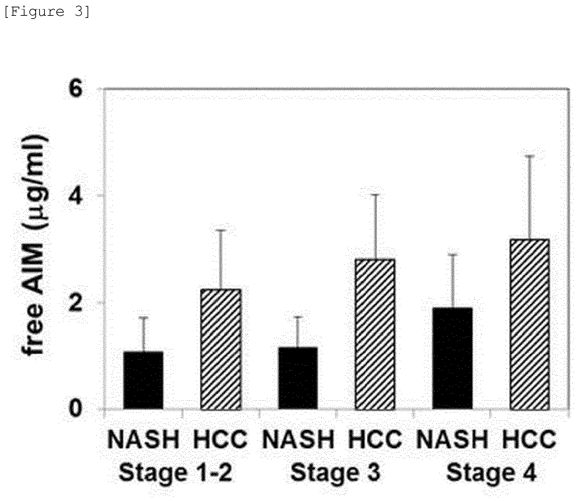

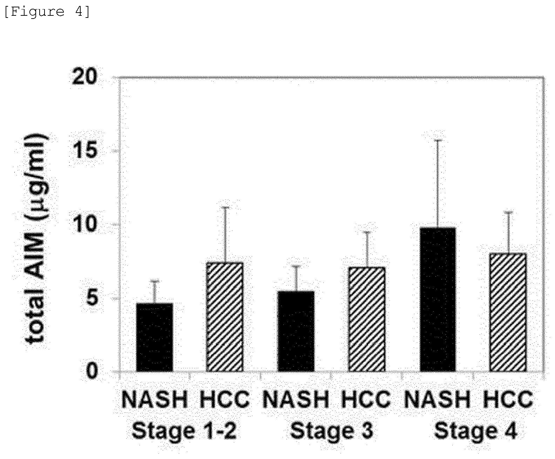

Free AIM was significantly increased in NASH liver cancer patients (HCC) compared to NASH patients for all stages (Figure 3). No significant difference in total AIM was seen between NASH patients and NASH liver cancer patients for all stages (Figure 4).

### Example 5: Comparison of Increase in Free AIM and Increase in Total AIM Along with Canceration

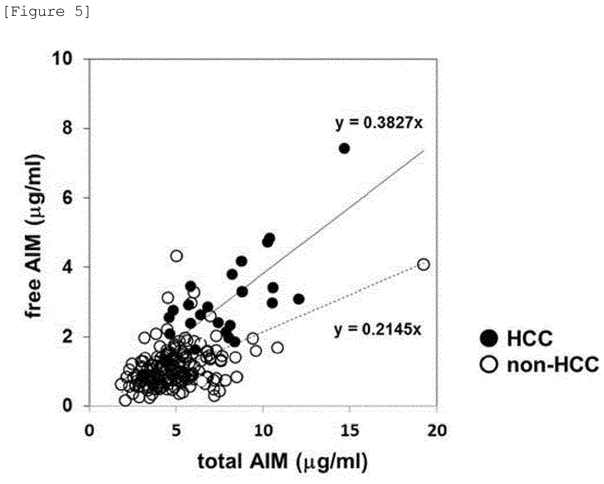

The aforementioned Human AIM ELISA kit (CY-8080, from CircuLex) that quantifies free AIM and the same ELISA which quantifies total AIM as used in Example 4 were employed to measure the concentration of free AIM and total AIM in the serum of 42 NAFL patient cases, 135 NASH patient cases, and 26 NASH liver cancer patient cases (HCC). Although free AIM increased along with the increase in total AIM, increase of free AIM in NASH liver cancer patients (HCC) (black circles) was significant compared to NAFL and NASH patients (collectively non-HCC) (white circles) (Figure 5).

### Example 6: ROC Analysis of NASH Patients and NASH Liver Cancer Patients, Progressed Fibrillization Cases, Hepatic Cirrhosis Cases

ROC analysis of the concentration of free AIM and total AIM in the serum of NASH patients (135 cases) and NASH liver cancer patients (HCC) (26 cases) was performed (See Table 1).

AUC was 0.927 for free AIM and 0.801 for total AIM (Table 1 (A)). Free AIM could discriminate liver cancer with higher precision.

It is known that the onset of liver cancer is more likely to occur as liver fibrillization progresses. ROC analysis of the concentration of free AIM and total AIM in the serum of progressed fibrillization cases at fibrillization stage 3 (F3) and stage 4 (F4) was performed. In the progressed fibrillization cases, AUC was 0.918 for free AIM and 0.709 for total AIM (Table 1 (B)). Free AIM could discriminate liver cancer with high precision also in progressed fibrillization cases, but precision with total AIM was low.

Hepatic cirrhosis cases with progressed fibrillization have the highest onset risk for liver cancer. ROC analysis of the concentration of free AIM and total AIM in the serum of hepatic cirrhosis cases at stage 4 (F4) was performed. In the hepatic cirrhosis cases, AUC was 0.869 for free AIM and 0.506 for total AIM (Table 1 (C)). Free AIM could discriminate liver cancer with high precision also in hepatic cirrhosis cases, but precision with total AIM was low.

**[Table 1]**

| | | (A) All cases | (B) Progressed fibrillization cases | (C) Hepatic cirrhosis cases |
|---|---|---|---|---|
| Number of cases | NASH | 135 | 37 | 7 |
| | NASH liver cancer | 26 | 22 | 12 |
| ROC-AUC | Free AIM | 0.927 | 0.981 | 0.869 |
| | Total AIM | 0.801 | 0.709 | 0.506 |

### Example 7: ROC Analysis in Non-HCC Patients and HCC Patients

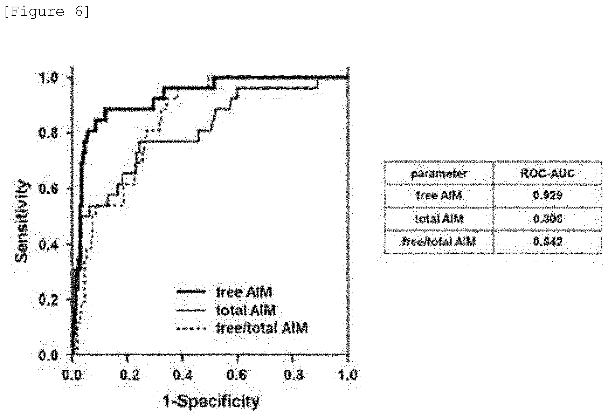

In NAFL and NASH patients (collectively non-HCC patients) (177 cases) and NASH liver cancer patients (HCC) (26 cases), ROC analysis was performed on the concentration of free AIM and total AIM in the serum as well as on the ratio of free AIM and total AIM concentrations. AUC was 0.929 for free AIM, 0.806 for total AIM, and 0.842 for free AIM/total AIM ratio, and free AIM could discriminate liver cancer patients with higher diagnosis efficiency (see Figure 6).

### Example 8: Analysis of Diagnosis Sensitivity and Specificity

The concentration of free AIM in blood was measured for NAFL and NASH patients (collectively non-HCC) (177 cases) and NASH liver cancer patients (HCC) (26 cases), diagnostic value of liver cancer by free AIM was investigated with the cutoff value at 1.6 µg/mL. As a result, patients who showed 1.6 µg/mL or more among NASH liver cancer patients (HCC) were 23 cases, and the diagnosis sensitivity was 0.885 (23/26). Moreover, patients who showed less than 1.6 µg/mL among patients who did not have liver cancer were 152 cases, and the diagnosis specificity was 0.859 (152/177) (Table 2).

**[Table 2]**

| | Non-HCC | HCC | total |
|---|---|---|---|
| Free AIM less than 1.6 µg/mL | 152 | 3 | 155 |
| Free AIM 1.6 µg/mL or more | 25 | 23 | 48 |
| Total | 177 | 26 | 203 |

### Example 9: Association with Tumor Marker

In 25 cases of NASH liver cancer patients, association of tumor markers (PIVKA-II and AFP) with the free AIM concentration, the total AIM concentration, and the ratio of free AIM and total AIM concentrations in blood was investigated. Patients were divided with the cutoff values for PIVKA-II and AFP as 40 mAU/mL and 20 ng/mL respectively, and the free AIM concentration, the total AIM concentration, and the ratio of free AIM and total AIM concentrations was analyzed for each patient. As a result, no significant difference was seen between the patient group who showed the cutoff value or more for each tumor marker and the patient group who showed less than the cutoff value in any of the free AIM concentration, the total AIM concentration, and the ratio of free AIM and total AIM concentrations (Table 3).

**[Table 3]**

| | PIVKA-II | | | AFP | | |
|---|---|---|---|---|---|---|
| | Less than 40 mAU | 40 mAU or more | P | Less than 20 ng | 20 ng or more | P |
| | Average (SD) | Average (SD) | | Average (SD) | Average (SD) | |
| Free AIM (mg/mL) | 2.83 (1.60) | 2.99 (1.20) | 0.406 | 2.73 (1.44) | 3.51 (1.05) | 0.121 |
| Total AIM (mg/mL) | 7.42 (3.19) | 7.70 (2.50) | 0.574 | 7.40 (2.90) | 8.11 (2.60) | 0.475 |
| Free/total ratio | 0.39 (0.13) | 0.38 (0.10) | 0.728 | 0.37 (0.12) | 0.44 (0.08) | 0.198 |

### Industrial Applicability

By employing the method of the present invention, liver cancer can be examined with superior sensitivity and specificity.

## Claims

1. A method for examining liver cancer comprising a step of quantifying an amount of free apoptosis inhibitor of macrophage (AIM) in a serum, plasma or whole blood sample derived from a test subject, wherein an increased amount compared to a control healthy individual indicates liver cancer, wherein free AIM means AIM protein that is not in complex with other binding partners such as IgM.

2. The method according to claim 1, wherein said liver cancer is liver cancer caused by non-alcoholic steatohepatitis (NASH).

3. The method according to claim 1 or 2, wherein said quantification is one by immunoassay.

4. The method according to claim 3, wherein said quantification is performed by contacting a biological sample with an antibody that specifically binds to free AIM.

5. The method according to claim 3 or claim 4, wherein said quantification is measured by ELISA.

6. The method according to any one of claims 2 to 5, **characterized in that** said quantification is performed without exposing the serum, blood plasma, or whole blood to a protein denaturing condition and to a reductive condition.

7. The method according to claim 1 further comprising a step of quantifying total AIM in a biological sample derived from a test subject.

8. The method according to claim 7 further comprising a step of calculating the ratio of free AIM and total AIM.

## Patentansprüche

1. Verfahren zur Untersuchung von Leberkrebs, das einen Schritt der Quantifizierung einer Menge an freiem Apoptoseinhibitor von Makrophagen (AIM) in einer Serum-, Plasma- oder Vollblutprobe umfasst, die von einer Testperson stammt, wobei eine erhöhte Menge im Vergleich zu einer gesunden Kontrollperson auf Leberkrebs hinweist, wobei freier AIM ein AIM-Protein bedeutet, das nicht mit anderen Bindungspartnern wie IgM in einem Komplex vorliegt.

2. Verfahren nach Anspruch 1, wobei der Leberkrebs durch nichtalkoholische Steatohepatitis (NASH) verursachter Leberkrebs ist.

3. Verfahren nach Anspruch 1 oder 2, wobei die Quantifizierung durch einen Immunoassay erfolgt.

4. Verfahren nach Anspruch 3, wobei die Quantifizierung durch Inkontaktbringen einer biologischen Probe mit einem Antikörper durchgeführt wird, der spezifisch an freien AIM bindet.

5. Verfahren nach Anspruch 3 oder Anspruch 4, wobei die Quantifizierung durch ELISA gemessen wird.

6. Verfahren nach einem der Ansprüche 2 bis 5, **dadurch gekennzeichnet, dass** die Quantifizierung durchgeführt wird, ohne das Serum, das Blutplasma oder das Vollblut einer proteindenaturierenden Bedingung und einer reduzierenden Bedingung auszusetzen.

7. Verfahren nach Anspruch 1, das ferner einen Schritt der Quantifizierung von Gesamt-AIM in einer biologischen Probe umfasst, die von einer Testperson stammt.

8. Verfahren nach Anspruch 7, das ferner einen Schritt der Berechnung des Verhältnisses von freiem AIM und Gesamt-AIM umfasst.

## Revendications

1. Procédé d'examen du cancer du foie comprenant une étape de quantification d'une quantité d'inhibiteur de l'apoptose des macrophages (AIM) libre dans un échantillon de sérum, de plasma ou de sang total dérivé d'un sujet de test, dans lequel une quantité accrue par rapport à un individu sain témoin indique un cancer du foie, dans lequel l'AIM libre signifie protéine d'AIM qui n'est pas en complexe avec d'autres partenaires de liaison tels que l'IgM.

2. Procédé selon la revendication 1, dans lequel ledit cancer du foie est un cancer du foie causé par la stéatohépatite non alcoolique (NASH).

3. Procédé selon la revendication 1 ou 2, dans lequel ladite quantification est une par dosage immunologique.

4. Procédé selon la revendication 3, dans lequel ladite quantification est effectuée par mise en contact d'un échantillon biologique avec un anticorps qui se lie spécifiquement à l'AIM libre.

5. Procédé selon la revendication 3 ou 4, dans lequel ladite quantification est mesurée par ELISA.

6. Procédé selon l'une quelconque des revendications 2 à 5, **caractérisé en ce que** ladite quantification est effectuée sans exposer le sérum, le plasma sanguin ou le sang total à un état dénaturant des protéines et à un état réductif.

7. Procédé selon la revendication 1 comprenant en outre une étape de quantification de l'AIM total dans un échantillon biologique issu d'un sujet de test.

8. Procédé selon la revendication 7 comprenant en outre une étape de calcul du rapport de l'AIM libre par rapport àl'AIM total.
